# FASCICULE DE BREVET EUROPEEN

(11) **EP 1 194 114 B1**
(45) Date de publication et mention de la délivrance du brevet: **23.11.2005**
(21) Numéro de dépôt: 00927325.1
(22) Date de dépôt: 10.05.2000
(51) Int. Cl.: A61K 7/06

(54) **COMPOSITION CONTENANT UN AGENT OPACIFIANT OU NACRANT ET AU MOINS DEUX ALCOOLS GRAS**
ZUSAMMENSETZUNG, DIE EIN TRÜBUNGS- ODER PERLGLANZMITTEL UND MINDESTENS ZWEI FETTALKOHOLE ENTHÄLT
COMPOSITION CONTAINING AN OPACIFIER OR AGENT PROVIDING PEARLY LUSTRE AND AT LEAST TWO FATTY ALCOHOLS

(30) Priorité: 23.06.1999 FR 9908029
(43) Date de publication de la demande: 10.04.2002
(73) Titulaire: L'OREAL, 75008 Paris (FR)
(72) Inventeur: DECOSTER, Sandrine, F-95210 Saint Gratien (FR); BEAUQUEY, Bernard, F-92110 Clichy (FR)
(74) Mandataire: Le Blainvaux Bellegarde, Françoise
(86) Numéro de dépôt international: PCT/FR2000/001257
(87) Numéro de publication internationale: WO 2001/000149

(56) Documents cités:
- WO-A-97/00668
- WO-A-99/13830
- WO-A-99/13844
- WO-A-99/62467
- WO-A-99/62492
- DATABASE CHEMICAL ABSTRACTS [en ligne] STN; abstract 111: 120 622, XP002134783 & JP 01 009916 A (KANEBO, LTD) 13 janvier 1989 (1989-01-13)

## Description

La présente invention a trait à une composition comprenant au moins un alcool gras linéaire et saturé à longue chaîne ayant 22 atomes de carbone, au moins un alcool gras linéaire et saturé à longue chaîne ayant 18 atomes de carbone et au moins un agent opacifiant et/ou nacrant. à une composition comprenant au moins une base tensioactive, au moins un alcool gras linéaire et saturé à longue chaîne ayant 22 atomes de carbone, au moins un alcool gras linéaire et saturé à longue chaîne ayant 18 atomes de carbone et au moins un agent opacifiant et/ou nacrant, à leur utilisation en tant qu'agent de nacrage, à une composition cosmétique comprenant dans un milieu cosmétiquement acceptable au moins une base tensioactive, au moins un alcool gras linéaire et saturé à longue chaîne ayant 22 atomes de carbone, au moins un alcool gras linéaire et saturé à longue chaîne ayant 18 atomes de carbone, au moins un agent opacifiant et/ou nacrant et au moins un agent de conditionnement des matières kératiniques. L'invention a également pour objet l'utilisation de ladite composition en tant qu'agent de suspension des agents de conditionnement insolubles.
Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.
On a déjà préconisé dans les compositions pour le lavage ou le soin des matières kératiniques telles que les cheveux l'utilisation d'agents de conditionnement, notamment insolubles, pour faciliter le démêlage des cheveux et pour leur communiquer douceur, brillance et souplesse.
Compte tenu du caractère insoluble de certains agents de conditionnement tels que par exemple les silicones ou les huiles, on cherche à maintenir les agents de conditionnement en dispersion régulière dans le milieu sans cependant faire chuter la viscosité et les propriétés détergentes et moussantes des compositions. Les silicones doivent également être véhiculées sur les matières kératiniques traitées en vue de leur conférer, suivant l'application, des propriétés de douceur, de brillance et de démêlage.
On sait également que les produits en particulier cosmétiques ayant un aspect ou un effet irisé, moiré ou métallisé, sont largement appréciés par les consommateurs pour leur aspect esthétique et donnant une apparence de richesse au produit. Les agents qui apportent cet effet sont des agents de nacrage comprenant généralement des cristaux qui restent dispersés dans les compositions et qui réfléchissent la lumière.

Il existe à ce jour peu de moyens pour maintenir efficacement en suspension les agents de conditionnement insolubles, car il s'agit là d'un problème difficile à résoudre ; à cet effet, on a déjà proposé l'utilisation des dérivés d'esters à longue chaîne ou des polysaccharides tels que la gomme de xanthane. Cependant, les dérivés d'esters à longue chaîne peuvent présenter des problèmes de cristallisation qui entraînent une évolution de la viscosité des compositions au cours du temps ; les agents gélifiants présentent également des inconvénients, à savoir, d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

On connaît également les dérivés d'éthers ou de thioéthers à longue chaîne tels que ceux décrits dans les demandes EP457688 et WO98/03155. Cependant, ces derniers agents opacifient les compositions sans apporter de nacrage aux compositions ou pas suffisamment.

On a déjà essayé d'améliorer le nacrage en ajoutant des agents épaississants et/ou d'autres agents nacrants, mais ainsi la viscosité devient trop importante et/ou la composition n'est plus stable.

La demanderesse a découvert, ce qui fait l'objet de l'invention, que l'utilisation d'au moins un alcool gras linéaire et saturé à longue chaîne ayant 22 atomes de carbone, d'au moins un alcool gras linéaire et saturé à longue chaîne ayant 18 atomes de carbone, le rapport alcool gras en C18/alcool gras en C22 étant supérieur à 0,15, permettait de nacrer et/ou améliorer le nacrage des compositions comprenant au moins une base tensioactive et au moins un agent opacifiant et/ou nacrant. De plus, cette association permet de stabiliser la viscosité en fonction de la température.

L'invention a donc pour objet des compositions comprenant au moins un alcool gras linéaire et saturé à longue chaîne ayant 22 atomes de carbone, au moins un alcool gras linéaire et saturé à longue chaîne ayant 18 atomes de carbone et au moins un agent opacifiant et/ou nacrant.

L'invention a également pour objet des compositions notamment comprenant au moins une base tensioactive, au moins un alcool gras linéaire et saturé à longue chaîne ayant 22 atomes de carbone, au moins un alcool gras linéaire et saturé à longue chaîne ayant 18 atomes de carbone et au moins un agent opacifiant et/ou nacrant, le rapport alcool gras en C18/alcool gras en C22 étant supérieur à 0,15.

Les compositions selon l'invention peuvent être utilisées comme base nacrante des compositions cosmétiques pour procurer un effet nacré supérieur à celui obtenu par l'agent opacifiant et/ou nacrant.

L'invention a encore pour objet l'utilisation d'au moins un alcool gras linéaire et saturé à longue chaîne ayant 22 atomes de carbone, d'au moins un alcool gras linéaire et saturé à longue chaîne ayant 18 atomes de carbone, le rapport alcool gras en C18/alcool gras en C22 étant supérieur à 0,15 et d'un agent opacifiant en tant que nouvel agent nacrant.

Les compositions présentent une très bonne homogénéité et une bonne stabilité du nacrage, ainsi qu'une viscosité satisfaisante pour l'application sur les matières kératiniques.

D'autres objets de l'invention apparaîtront à la lecture de la description et des exemples qui suivent.

L'alcool gras ayant 22 atomes de carbone est notamment commercialisé sous forme d'un mélange d'alcools gras. Plus particulièrement, le mélange comprend au moins 70% en poids d'alcool en C22 par rapport au poids total du mélange d'alcools gras.

De tels mélanges d'alcools gras sont notamment les produits commercialisés sous la dénomination NAFOL 1822 C par la société CONDEA qui contient environ 74-78% de C22 ou le produit commercialisé sous la dénomination NAFOL 2298 par la société CONDEA qui contient environ 98% d'alcool en C22.

Selon l'invention, l'alcool gras linéaire et saturé ayant 22 atomes de carbone peut représenter de 0,5% à 10 % en poids, de préférence de 0,5 % à 5 % en poids, et encore plus préférentiellement de 0,5 % à 3 % en poids, du poids total de la composition finale.

Selon l'invention, l'alcool gras linéaire et saturé ayant 18 atomes de carbone peut représenter de 0,3% à 10 % en poids, de préférence de 0,5 % à 5 % en poids, et encore plus préférentiellement de 0,5 % à 3 % en poids, du poids total de la composition finale.

Le rapport alcool gras en C18/alcool gras en C22 supérieur à 0,15, est généralement compris entre 0,2 et 20, notamment entre 0,25 et 10, et de préférence entre 0,3 et 5.

Les agents nacrants et/ou opacifiants utilisables selon l'invention peuvent être choisis parmi :
A) les dialkyléthers gras solides à une température inférieure ou égale à environ 30°C tels que par exemple les dialkyléthers de formule (I) :

   R-O-R' (I)

   dans laquelle:
   R et R', identiques ou différents, désignent un radical alkyle, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ
      Plus particulièrement R et R' sont identiques.

   De façon préférentielle, R et R' désignent un radical stéaryle.
   Les dialkyléthers utilisables dans les compositions selon l'invention sont insolubles dans l'eau, c'est à dire qu'ils sont insolubles dans l'eau à une concentration supérieure ou égale à 0,1% en poids dans l'eau à 25°C.
   Ces composés peuvent être préparés selon le procédé décrit dans la demande de brevet DE 41 27 230.
   Un distéaryléther utilisable dans le cadre de la présente invention, est notamment vendu sous la dénomination CUTINA STE par la société HENKEL.
B) les alcools ayant de 27 à 48 atomes de carbone et comportant un ou deux groupements éther et/ou thioéther ou sulfoxyde répondant à la formule (II) :

   R1-X-[C2H3(OH)]-CH2-Y-R2 (II)

   dans laquelle
   R1 et R2 désignent indépendamment l'un de l'autre, des groupements alkyles linéaires en C12 à C24;
   X désigne un atome d'oxygène, un atome de soufre, un groupement sulfoxyde ou méthylène ;
   Y désigne un atome d'oxygène, un atome de soufre, un groupement sulfoxyde ou méthylène ;
   dans le cas où Y désigne un groupement méthylène la somme du nombre d'atomes de carbone présents dans les groupements R1 et R2 a une valeur variant de 24 à 44 et de préférence de 28 à 40 inclus ;
   lorsque Y ne désigne pas un groupement méthylène, la somme des atomes de carbone présents dans les groupements R1 et R2 a une valeur variant de 24 à 44 et de préférence de 28 à 40 inclus ;
   lorsque X ou Y désigne sulfoxyde, Y ou X ne désigne pas soufre.
   Les composés de formules (II) utilisés de préférence conformément à l'invention, sont ceux pour lesquels X désigne oxygène, Y désigne méthylène, et R1 et R2 désignent des radicaux ayant 12 à 22 atomes de carbone.
   Ces composés peuvent être préparés selon le brevet EP 457 688.
C) les dérivés acylés comportant au moins une chaîne grasse ayant de 8 à 30 atomes de carbone. Par dérivé acylés, on comprend les composés comprenant au moins un groupe RC(=O)-, R désignant une chaîne grasse ayant de 8 à 30 atomes de carbone.

Parmi ces composés, on peut notamment citer le monostéarate d'éthylèneglycol, le distéarate d'éthylèneglycol.

Selon l'invention, l'agent nacrant et/ou opacifiant peut représenter de 0,5 % à 15 % en poids, de préférence de 1 % à 5 % en poids, du poids total de la composition finale.

Les compositions de l'invention contiennent en outre au moins une base tensioactive qui est généralement présente en une quantité comprise entre 1% et 60% en poids environ, de préférence entre 3% et 40% et encore plus préférentiellement entre 5% et 30%, par rapport au poids total de la composition.

La base tensioactive est constituée de tensioactifs pouvant être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkylamido(C₆-C₂₄) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s) ou zwittérionique(s) :

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits commercialisés sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

R₂ -CONHCH₂CH₂ -N(R₃)(R₄)(CH₂COO-) (2)

dans laquelle :
R₂ désigne un radical alkyle d'un acide R₂-COOH présent dans l'huile de coprah hydrolysée, un radical heptyle, nonyle ou undécyle,
R₃ désigne un groupement bêta-hydroxyéthyle et
R₄ un groupement carboxyméthyle ;
et

R₂.-CONHCH₂CH₂-N(B)(C) (3)

dans laquelle:
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R₂, désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate commercialisé sous la dénomination commerciale MIRANOL C2M concentré par la Société RHODIA CHIMIE.

### (iv) Tensioactifs cationiques :

Parmi les tensioactifs cationiques, dont la nature ne revet pas dans le cadre de la présente invention de caractère critique, on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Selon une variante préférée de l'invention, les compositions cosmétiques peuvent également contenir des agents de conditionnement des matières kératiniques.

L'invention a donc également pour objet de nouvelles compositions cosmétiques en particulier de lavage et de conditionnement moussantes comprenant dans un milieu cosmétiquement acceptable une base tensioactive, au moins un agent de conditionnement, au moins un alcool gras linéaire et saturé à longue chaîne ayant 22 atomes de carbone, au moins un alcool gras linéaire et saturé à longue chaîne ayant 18 atomes de carbone et au moins un agent opacifiant et/ou nacrant.

Les compositions ainsi préparées possèdent en outre, de bonnes propriétés détergentes et moussantes et confèrent aux matières kératiniques, notamment aux cheveux et/ou à la peau, une grande douceur.

Ces compositions, lorsqu'elles sont appliquées sur les cheveux, possèdent, en plus de leurs propriétés lavantes, des propriétés de conditionnement des cheveux, c'est-à-dire que les cheveux traités sont lisses, se démêlent facilement, sont doux au toucher. Les cheveux ont un aspect naturel et non chargé.

Les compositions selon l'invention contenant des agents de conditionnement sont stables : en particulier, il ne se produit aucun relargage des agents de conditionnement ou d'épaississement incontrôlé de la composition au cours du temps. Les compositions présentent enfin une texture non filante et fondante. La mousse se rince facilement.

Un autre objet de l'invention est constitué par le procédé de lavage et de conditionnement mettant en oeuvre de telles compositions.

L'invention a encore pour objet l'utilisation d'au moins un alcool gras linéaire et saturé à longue chaîne ayant 22 atomes de carbone, d'au moins un alcool gras linéaire et saturé à longue chaîne ayant 18 atomes de carbone et d'au moins un agent opacifiant et/ou nacrant comme agent de mise en suspension d'un agent de conditionnement insoluble dans une composition cosmétique en particulier de lavage et de conditionnement moussante contenant, dans un milieu aqueux cosmétiquement acceptable, une base tensioactive.

Lorsque la composition contient au moins un agent de conditionnement, ils sont généralement choisis parmi les huiles de synthèse telles que les poly-α-oléfines, les huiles fluorées, les cires fluorées, les gommes fluorées, les esters d'acides carboxyliques, les polymères cationiques, les silicones, les huiles minérales, végétales ou animales, les céramides, les pseudocéramides et leurs mélanges.

Les polyoléfines sont de préférence des poly-α-oléfines et en particulier :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les polyisobutènes vendus sous le nom de PERMETHYL 99 A, 101 A, 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par :
- les hydrocarbures, tels que l'hexadécane et l'huile de paraffine ;

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium).
On peut également utiliser les polymères qui sont constitués de motifs récurrents répondant à la formule : 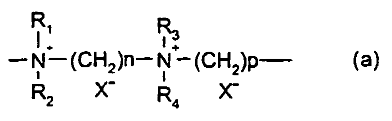 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Les silicones utilisables conformément à l'invention sont en particulier des polyorganosiloxanes insolubles dans la composition et peuvent se présenter sous forme d'huiles, de cires, de résines ou de gommes.

Les silicones insolubles dans l'eau sont insolubles dans l'eau à une concentration supérieure ou égale à 0,1% en poids dans l'eau à 25°C, c'est à dire qu'elles ne forment pas une solution isotrope transparente.

La viscosité des silicones est par exemple mesurée à 25°C selon la norme ASTM 445 Appendice C. Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 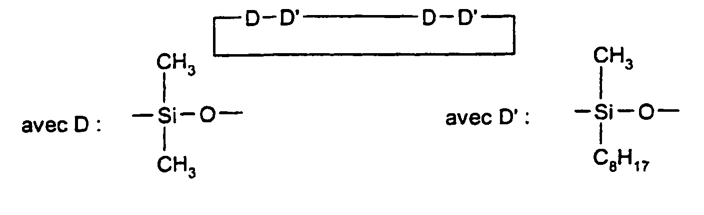
   On peut également citer les mélanges de silicones cycliques avec des composés organiques dérivés du silicium, tels que le mélange d'octaméthylcyclotétrasiloxane et de tétratriméthylsilylpentaérythritol (50150) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 000 ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles MIRASIL DPDM de RHODIA CHIMIE ;
. les huiles des séries RHODORSIL 70 633 et 763 de RHODIA CHIMIE ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanesiméthylvinyisiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthylitriméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (IX) : 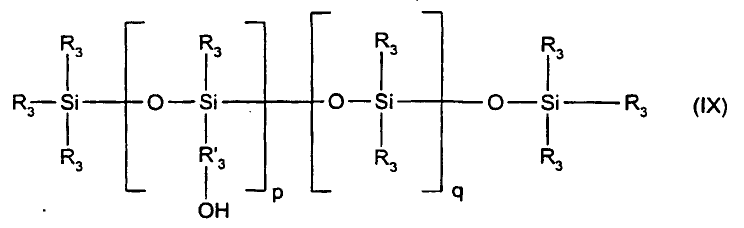 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans le brevet US-A-4957732 et répondant à la formule (X) : 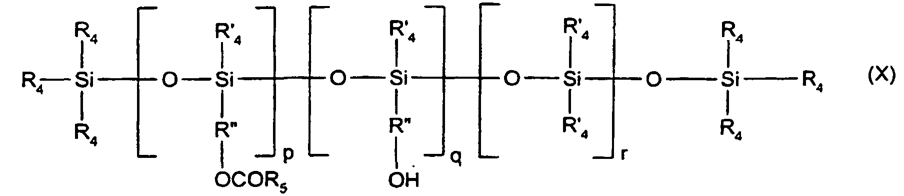 dans laquelle :
   R₄ désigne un groupement méthyle, phényle, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀;
   R" désigne un radical alkylène hydrocarboné divalent, linéaire ou ramifié en C₂-C₁₈;
   r est compris entre 1 et 120 indus ;
   p est compris entre 1 et 30 ;
   q est égal à 0 ou est inférieur à 0,5 p, p + q étant compris entre 1 et 30 ; les polyorganosiloxanes de formule (VI) peuvent contenir des groupements : 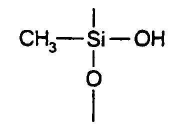 dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicataire à partir du mélange de monomères constitué par :
a) 50 à 90% en poids d'acrylate de tertiobutyle ;
b) 0 à 40% en poids d'acide acrytique ;
c) 5 à 40% en poids de macromère siliconé de formule : 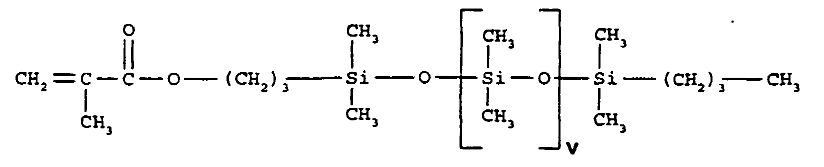 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polysiloxanes à groupements aminés tels que les amodiméthicones ou les triméthylsilylamodiméthicones ;

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

Selon l'invention, les agents conditionneurs peuvent représenter de 0,001 % à 10 % en poids, de préférence de 0,005 % à 5 % en poids, et encore plus préférentiellement de 0,01 % à 3 % en poids, du poids total de la composition finale.

Le milieu cosmétiquement acceptable est de préférence constitué par de l'eau ou un mélange d'eau et de solvants cosmétiquement ou dermatologiquement acceptables tels que des monoalcools, des polyalcools, des éthers de glycol qui peuvent être utilisés seuls ou en mélange.

On peut citer plus particulièrement les alcools inférieurs tels que l'éthanol, l'isopropanol, les polyalcools tels que le diéthylèneglycol, la glycérine, les éthers de glycol, les alkyléthers de glycol ou de diéthylèneglycol.

La composition de l'invention peut également contenir au moins un additif choisi parmi les séquestrants, les adoucissants, les modificateurs de mousse, les colorants, d'autres agents nacrants, les agents hydratants, les agents antipelliculaires ou anti-séborrhéiques, d'autres agents de mise en suspension, les acides gras à chaînes linéaires ou ramifiées en C₁₆-C₄₀, les hydroxyacides, les électrolytes, les épaississants, les esters d'acides gras, les parfums, les conservateurs, les filtres solaires, les protéines, les vitamines et provitamines, les polymères, et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions conformes à l'invention peuvent être utilisées pour le traitement des matières kératiniques telles que les cheveux, la peau, les cils, les sourcils, les ongles, les lèvres, le cuir chevelu et plus particulièrement les cheveux.

Les compositions peuvent également être utilisées pour le lavage et le nettoyage des matières kératiniques telles que les cheveux et la peau.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

En particulier, les compositions selon l'invention sont des compositions détergentes moussantes telles que des shampooings, des gels-douche et des bains moussants. Dans ce mode de réalisation de l'invention, les compositions comprennent une base tensioactive lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques décrits ci-dessus.

La quantité minimale de base lavante est celle juste suffisante pour conférer à la composition finale un pouvoir moussant et/ou détergent satisfaisant.

Ainsi, selon l'invention, la base lavante peut représenter de 4 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement cosmétique des matières kératiniques telles que les cheveux consistant à appliquer sur ceux-ci une composition telle que définie précédemment puis à effectuer éventuellement un rinçage à l'eau, après un éventuel temps de pause.

L'invention va être maintenant plus complètement illustrée à l'aide des exemples suivants qui ne sauraient être considérés comme la limitant aux modes de réalisation décrits. Dans ce qui suit, MA signifie Matière Active.

### EXEMPLE 1

On a préparé deux shampooings de compositions suivantes :
La composition A est selon l'invention, la composition B est une composition comparative de l'art antérieur.

| | A(invention) | B |
|---|---|---|
| - Lauryléther sulfate de sodium oxyéthyléné à 2,2 moles d'oxyde d'éthylène en solution aqueuse à 70% de MA | 14 gMA | 14 gMA |
| - Tensioactif dérivé d'imidazoline (MIRANOL C2M conc de RHODIA CHIMIE) | 4 gMA | 4 g MA |
| - Diméthicone (MIRASIL DM 500000 de RHODIA CHIMIE) | 2,6 g | 2,6 g |
| - Gomme de guar modifiée par du chlorure de 2.3-époxypropyl triméthylammonium (JAGUAR C13 S de RHODIA CHIMIE) | 0,2 g | 0,2 g |
| - Mélange d'alcool stéarylique (10%) et de distéaryléther (90%) | 4 g | 4 g |
| - Alcool gras en C18 (98%) | 0,25 g | 1 g |
| - Alcool gras en C22 (76%) (NAFOL 1822 C de CONDEA) | 0,75 gMA | - |
| - Cétostéaryl(50/50)sulfate de sodium | 0,75 g | 0,75 g |
| - Alcool stéarylique oxyéthyléné 10 moles d'oxyde d'éthylène (BRIJ 76 de ICI) | 0,8 g | 0,8 g |
| - Conservateurs, parfum | qs | qs |
| - Acide citrique, 1H2O qs | pH 5 | pH 5 |
| - Eau déminéralisée qsp | 100 g | 100 g |

La viscosité des compositions est mesurée à l'aide d'un Viscotesteur VT550 commercialisé par RHEO (mobile MV din B) à une vitesse de cisaillment de 10s⁻¹ et à une température de 25°C.

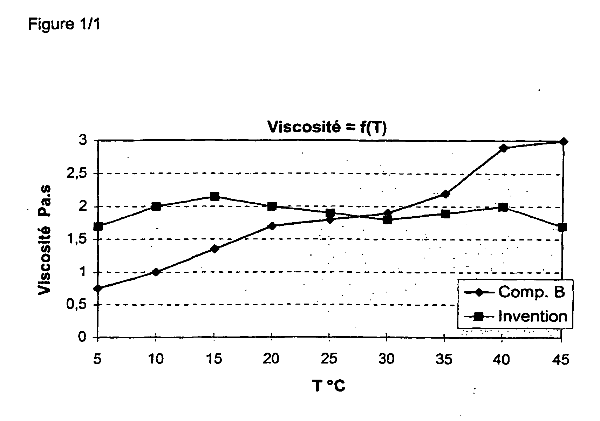

Les résultats sont donnés dans la figure 1 annexée.
La viscosité de la composition A selon l'invention est moins dépendante de la température que celle de la composition B de l'art antérieur (WO98/03155), ces deux shampooings ayant une viscosité comparable à température ambiante (25°C).
De plus, la composition selon l'invention a un effet blanc nacré plus important que celui de la composition B.

### EXEMPLES 2 et 3

| | Ex. 2 | Ex. 3 |
|---|---|---|
| Lauryléthersulfate de sodium (C12/C14 à 70/30) à 2.2 moles d'oxyde d'éthylène | 14.0 g MA | 14.0 g M.A. |
| Cocoyl bétaïne en solution aqueuse à 30% M.A. | 2.4 g MA | |
| Cocoyl amidopropyl bétaïne en solution aqueuse à 38% M.A. | | 2.7g MA |
| Hydroxyéthylcellulose réticulée par l'épichlorhydrine et quatemisée par la triméthylamine, vendue sous la dénomination JR 400 par la société UNION CARBIDE | 0.3 g | 0.3g |
| Polydiméthylsiloxane vendu sous la dénomination MIRASIL DM 500 000 par RHODIA CHIMIE | 2.0 g | 2.5g |
| Distéarate de glycol | 2.0 g | |
| Mélange 1-(hexadécyloxy) 2-octadécanol / Alcool cétylique | | 2.2 g |
| Alcool cétylstéarylique à 98% en alcool stéarylique | 0.5 g | 0.4 g |
| Mélange d'alcools gras à 76% d'alcool béhénique commercialisé sous la dénomination NAFOL 1822 C par la société CONDEA | 0.5 g | 0.6 g |
| Monoisopropylamide d'acides de coprah | 0.7 g | 0.7 g |
| Acide citrique q.s. pH | 5.5 | 5.5 |
| Parfum, conservateurs | q.s. | q.s. |
| Eau déminéralisée q.s.p. | 100.0 g | 100.0 g |

## Revendications

1. Composition cosmétique **caractérisée en ce qu'**elle comprend dans un milieu cosmétiquement acceptable au moins une base tensioactive, au moins un alcool gras linéaire et saturé à longue chaîne ayant 22 atomes de carbone, au moins un alcool gras linéaire et saturé à longue chaîne ayant 18 atomes de carbone et au moins un agent opacifiant et/ou nacrant, le rapport alcool gras en C18/alcool gras en C22 étant supérieur à 0,15.

2. Composition selon la revendication 1, **caractérisée par le fait que** les agents nacrants et/ou opacifiants sont choisis parmi :
A) les dialkyléthers gras solides à une température inférieure ou égale à environ 30°C tels que par exemple les dialkyléthers de formule (I) :
R-O-R' (I)
dans laquelle :
R et R', identiques ou différents, désignent un radical alkyle, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ
Plus particulièrement R et R' sont identiques.
B) les alcools ayant de 27 à 48 atomes de carbone et comportant un ou deux groupements éther et/ou thioéther ou sulfoxyde répondant à la formule (II) :
R1-X-[C2H3(OH)]-CH2-Y-R2 (II)
dans laquelle
R1 et R2 désignent indépendamment l'un de l'autre, des groupements alkyles linéaires en C12 à C24;
X désigne un atome d'oxygène, un atome de soufre, un groupement sulfoxyde ou méthylène ;
Y désigne un atome d'oxygène, un atome de soufre, un groupement sulfoxyde ou méthylène ;
dans le cas où Y désigne un groupement méthylène la somme du nombre d'atomes de carbone présents dans les groupements R1 et R2 a une valeur variant de 24 à 44 et de préférence de 28 à 40 inclus ;
lorsque Y ne désigne pas un groupement méthylène, la somme des atomes de carbone présents dans les groupements R1 et R2 a une valeur variant de 24 à 44 et de préférence de 28 à 40 inclus ;
lorsque X ou Y désigne sulfoxyde, Y ou X ne désigne pas soufre.
C) les dérivés acylés.

3. Composition selon l'une quelconque des revendications 1 ou 2, **caractérisée en ce que** les agents nacrants et/ou opacifiant sont choisis parmi :
A) le distéaryléther,
B) les composés de formules (II) pour lesquels X désigne oxygène, Y désigne méthylène, et R1 et R2 désignent des radicaux ayant 12 à 22 atomes de carbone,
C) le distéarate d'éthylèneglycol.

4. Composition selon l'une quelconque des revendications 1 à 3, **caractérisée en ce que** l'alcool gras linéaire et saturé ayant 22 atomes de carbone représente de 0,5% à 10 % en poids, de préférence de 0,5 % à 5 % en poids, et encore plus préférentiellement de 0,5 % à 3 % en poids, du poids total de la composition finale.

5. Composition selon l'une quelconque des revendications 1 à 4, **caractérisée en ce que** l'alcool gras linéaire et saturé ayant 18 atomes de carbone représente de 0,3% à 10 % en poids, de préférence de 0,5 % à 5 % en poids, et encore plus préférentiellement de 0,5 % à 3 % en poids, du poids total de la composition finale.

6. Composition selon l'une quelconque des revendications 1 à 5, **caractérisée en ce que** le rapport alcool gras en C18/alcool gras en C22 est compris entre 0,2 et 20, notamment entre 0,25 et 10, et de préférence entre 0,3 et 5.

7. Composition selon l'une quelconque des revendications 1 à 6, **caractérisée en ce que** l'agent opacifiant et/ou nacrant représente de 0,5% à 15% en poids, de préférence de 1% à 5% en poids du poids total de la composition finale.

8. Composition selon l'une quelconque des revendications 1 à 7, **caractérisée en ce que** la base tensioactive est présente entre 1% et 60% en poids, de préférence entre 3% et 40% et encore plus préférentiellement entre 5% et 30%, par rapport au poids total de la composition.

9. Composition selon l'une quelconque des revendications précédentes, **caractérisée en ce qu'**elle comprend en outre au moins un agent de conditionnement.

10. Composition selon la revendication précédente, **caractérisée en ce que** l'agent de conditionnement est choisi parmi les poly-α-oléfines, les huiles fluorées, les cires fluorées, les gommes fluorées, les esters d'acides carboxyliques, les silicones, les polymères cationiques, les huiles minérales, végétales ou animales, les céramides, les pseudocéramides, et leurs mélanges.

11. Composition selon la revendication précédente, **caractérisée en ce** les agents conditionneurs représentent de 0,001 % à 10 % en poids, de préférence de 0,005 % à 5 % en poids, et encore plus préférentiellement de 0,01 % à 3 % en poids, du poids total de la composition.

12. Composition selon l'une quelconque des revendications précédentes, **caractérisée par le fait qu'**elle se présente sous forme de gel, de lait, de crème, de lotion plus ou moins épaissie ou de mousse.

13. Composition selon l'une quelconque des revendications précédentes, **caractérisée par le fait qu'**elle constitue une composition détergente moussante telles que des shampooings, des gels-douche et des bains moussants.

14. Utilisation d'au moins un alcool gras linéaire et saturé à longue chaîne ayant 22 atomes de carbone, d'au moins un alcool gras linéaire et saturé à longue chaîne ayant 18 atomes de carbone et d'au moins un agent opacifiant et/ou nacrant , le rapport alcool gras en C18/alcool gras en C22 étant supérieur à 0,15 comme agent de mise en suspension d'un agent de conditionnement insoluble dans une composition cosmétique en particulier de lavage et de conditionnement moussante contenant, dans un milieu aqueux cosmétiquement acceptable, une base tensioactive.

15. Utilisation d'au moins un alcool gras linéaire et saturé à longue chaîne ayant 22 atomes de carbone, d'au moins un alcool gras linéaire et saturé à longue chaîne ayant 18 atomes de carbone, le rapport alcool gras en C18/alcool gras en C22 étant supérieur à 0,15 pour nacrer et/ou améliorer le nacrage des compositions comprenant au moins un agent opacifiant et/ou nacrant et éventuellement au moins une base tensioactive.

16. Utilisation d'au moins un alcool gras linéaire et saturé à longue chaîne ayant 22 atomes de carbone, d'au moins un alcool gras linéaire et saturé à longue chaîne ayant 18 atomes de carbone, le rapport alcool gras en C18/alcool gras en C22 étant supérieur à 0,15 et d'un agent opacifiant et/ou nacrant en tant qu'agent nacrant.

17. Procédé de traitement cosmétique des matières kératiniques en particulier des cheveux, **caractérisé par le fait qu'**il consiste à appliquer sur lesdites matières une composition telle que définie selon l'une quelconque des revendications 1 à 13 puis à effectuer éventuellement un rinçage à l'eau.

## Patentansprüche

1. Kosmetische Zusammensetzung, **dadurch gekennzeichnet, dass** sie in einem kosmetisch akzeptablen Medium mindestens eine grenzflächenaktive Basisformulierung, mindestens einen geradkettigen und gesättigten, langkettigen Fettalkohol mit 22 Kohlenstoffatomen, mindestens einen geradkettigen und gesättigten, langkettigen Fettalkohol mit 18 Kohlenstoffatomen und mindestens ein Trübungsmittel und/oder Perlglanzmittel enthält, wobei das Verhältnis C18-Fettalkohol/C22-Fettalkohol über 0,15 liegt.

2. Zusammensetzung nach Anspruch 1, **dadurch gekennzeichnet, dass** die Perlglanzmittel und/ oder Trübungsmittel unter den folgenden Verbindungen ausgewählt sind:
A) Bei einer Temperatur von etwa 30°C oder darunter festen Dialkylethern mit Fettkette, beispielsweise Dialkylethern der folgenden Formel (I):
R-O-R' (I)
worin bedeuten:
die Gruppen R und R', die gleich oder verschieden sind, eine gesättigte oder ungesättigte, geradkettige oder verzweigte Alkylgruppe mit 12 bis 30 Kohlenstoffatomen und vorzugsweise 14 bis 24 Kohlenstoffatomen, wobei R und R' so ausgewählt sind, dass die Verbindung der Formel (I) bei einer Temperatur von höchstens etwa 30°C fest ist.
Die Gruppen R und R' sind insbesondere identisch.
B) Alkoholen mit 27 bis 48 Kohlenstoffatomen, die eine oder zwei Ether- und/oder Thioethergruppen oder Sulfoxidgruppen und der folgenden Formel (II) entsprechen:
R1-X-[C2H3(OH)]-CH2-Y-R2 (II)
worin bedeuten
R1 und R2 unabhängig voneinander geradkettige Alkylgruppen mit 12 bis 24 Kohlenstoffatomen,
X ein Sauerstoffatom, ein Schwefelatom, eine Sulfoxidgruppe oder eine Methylengruppe,
Y ein Sauerstoffatom, ein Schwefelatom, eine Sulfoxidgruppe oder eine Methylengruppe,
falls Y eine Methylengruppe bedeutet, liegt die Summe der Anzahl der Kohlenstoffatome in den Gruppen R1 und R2 im Bereich von 24 bis 44 und vorzugsweise 28 bis 40;
wenn Y keine Methylengruppe ist, liegt die Summe der Kohlenstoffatome in den Gruppen R1 und R2 im Bereich von 24 bis 44 und vorzugsweise im Bereich von 28 bis 40;
wenn X oder Y Sulfoxid bedeutet, bedeuten X oder Y kein Schwefelatom.
C) Acylierten Derivaten.

3. Zusammensetzung nach einem der Ansprüche 1 oder 2, **dadurch gekennzeichnet, dass** die Perlglanzmittel und/oder Trübungsmittel ausgewählt sind unter:
A) dem Distearylether,
B) Verbindungen der Formel (II), worin X Sauerstoff, Y Methylen und R1 und R2 Gruppen mit 12 bis 22 Kohlenstoffatomen bedeuten,
C) Ethylenglykoldistearat.

4. Zusammensetzung nach einem der Ansprüche 1 bis 3, **dadurch gekennzeichnet, dass** der geradkettige und gesättigte Fettalkohol mit 22 Kohlenstoffatomen 0,5 bis 10 Gew.-%, vorzugsweise 0,5 bis 5 Gew.-% und noch bevorzugter 0,5 bis 3 Gew.-% des Gesamtgewichts der fertigen Zusammensetzung ausmacht.

5. Zusammensetzung nach einem der Ansprüche 1 bis 4, **dadurch gekennzeichnet, dass** der geradkettige und gesättigte Fettalkohol mit 18 Kohlenstoffatomen 0,3 bis 10 Gew.-%, vorzugsweise 0,5 bis 5 Gew.-% und noch bevorzugter 0,5 bis 3 Gew.-% des Gesamtgewichts der fertigen Zusammensetzung ausmacht.

6. Zusammensetzung nach einem der Ansprüche 1 bis 5, **dadurch gekennzeichnet, dass** das Verhältnis C18-Fettalkohol/C22-Fettalkohol im Bereich von 0,2 bis 20, insbesondere 0,25 bis 10 und vorzugsweise 0,3 bis 5 liegt.

7. Zusammensetzung nach einem der Ansprüche 1 bis 6, **dadurch gekennzeichnet, dass** das Trübungsmittel und/oder Perlglanzmittel 0,5 bis 15 Gew.-% und vorzugsweise 1 bis 5 Gew.-% des Gesamtgewichts der fertigen Zusammensetzung ausmacht.

8. Zusammensetzung nach einem der Ansprüche 1 bis 7, **da** **durch gekennzeichnet, dass** die grenzflächenaktive Basisformulierung 1 bis 60 Gew.-%, vorzugsweise 3 bis 40 Gew.-% und noch bevorzugter 5 bis 30 Gew.-%, bezogen auf das Gesamtgewicht der Zusammensetzung, ausmacht.

9. Zusammensetzung nach einem der vorhergehenden Ansprüche, **dadurch gekennzeichnet, dass** sie ferner mindestens ein Konditioniermittel enthält.

10. Zusammensetzung nach dem vorhergehenden Anspruch, **dadurch gekennzeichnet, dass** das Konditioniermittel unter den Poly-α-olefinen, fluorierten Ölen, fluorierten Wachsen, fluorierten Gummis, Carbonsäureestern, Siliconen, kationischen Polymeren, Mineralölen, pflanzlichen Ölen oder tierischen Ölen, Ceramiden, Pseudoceramiden und deren Gemischen ausgewählt ist.

11. Zusammensetzung nach dem vorhergehenden Anspruch, **dadurch gekennzeichnet, dass** die Konditioniermittel 0,001 bis 10 Gew.-%, vorzugsweise 0,005 bis 5 Gew.-% und noch bevorzugter 0,01 bis 3 Gew.-% des Gesamtgewichts der Zusammensetzung ausmachen.

12. Zusammensetzung nach einem der vorhergehenden Ansprüche, **dadurch gekennzeichnet, dass** sie als Gel, Milch, Creme, mehr oder weniger dickflüssige Lotion oder Schaum vorliegt.

13. Zusammensetzung nach einem der vorhergehenden Ansprüche, **dadurch gekennzeichnet, dass** es sich um schäumende, reinigende Zusammensetzungen handelt, beispielsweise Haarwaschmittel, Duschgels oder Schaumbäder.

14. Verwendung mindestens eines geradkettigen und gesättigten, langkettigen Fettalkohols mit 22 Kohlenstoffatomen, mindestens eines geradkettigen und gesättigten, langkettigen Fettalkohols mit 18 Kohlenstoffatomen und mindestens eines Trübungsmittels und/oder Perlglanzmittels, wobei das Verhältnis C18-Fettalkohol/C22-Fettalkohol über 0,15 liegt, als Suspendiermittel für ein Konditioniermittel, das in einer kosmetischen Zusammensetzung, insbesondere einer Zusammensetzung zur Reinigung und Konditionierung, die Schaum bildet und in einem kosmetisch akzeptablen wässerigen Medium eine grenzflächenaktive Basisformulierung enthält, unlöslich ist.

15. Verwendung mindestens eines geradkettigen und gesättigten, langkettigen Fettalkohols mit 22 Kohlenstoffatomen und mindestens eines geradkettigen und gesättigten, langkettigen Fettalkohols mit 18 Kohlenstoffatomen, wobei das Verhältnis C18-Fettalkohol/C22-Fettalkohol über 0,15 liegt, um Zusammensetzungen, die mindestens ein Trübungsmittel und/oder Perlglanzmittel und gegebenenfalls mindestens eine grenzflächenaktive Reinigungsformulierung enthalten, Perlglanz zu geben und/oder ihren Perlglanz zu verbessern.

16. Verwendung mindestens eines geradkettigen und gesättigten, langkettigen Fettalkohols mit 22 Kohlenstoffatomen, mindestens eines geradkettigen und gesättigten, langkettigen Fettalkohols mit 18 Kohlenstoffatomen, wobei das Verhältnis C 18-Fettalkohol/ C22-Fettalkohol über 0,15 liegt, und eines Trübungsmittels und/oder Perlglanzmittels als Perlglanzmittel.

17. Verfahren zur kosmetischen Behandlung von Keratinsubstanzen und insbesondere Haaren, **dadurch gekennzeichnet, dass** es darin besteht, auf die Keratinsubstanzen eine Zusammensetzung nach einem der Ansprüche 1 bis 13 aufzutragen und anschließend gegebenenfalls mit Wasser zu spülen.

## Claims

1. Cosmetic composition, **characterized in that** it comprises, in a cosmetically acceptable medium, at least one surfactant base, at least one saturated linear fatty alcohol with a long chain containing 22 carbon atoms, at least one saturated linear fatty alcohol with a long chain containing 18 carbon atoms and at least one opacifier and/or pearlescent agent, the C18 fatty alcohol/C22 fatty alcohol ratio being greater than 0.15.

2. Composition according to Claim 1, **characterized in that** the pearlescent agents and/or opacifiers are chosen from:
A) fatty dialkyl ethers which are solid at a temperature of less than or equal to about 30°C, such as, for example, the dialkyl ethers of formula (I):
R-O-R' (I)
in which:
R and R', which may be identical or different, denote a saturated or unsaturated, linear or branched alkyl radical comprising from 12 to 30 carbon atoms and preferably from 14 to 24 carbon atoms, R and R' being chosen such that the compound of formula (I) is solid at a temperature of less than or equal to about 30°C; more particularly, R and R' are identical;
B) alcohols containing from 27 to 48 carbon atoms and comprising one or two ether and/or thioether or sulphoxide groups corresponding to formula (II):
R1-X-[C2H3(OH)]-CH2-Y-R2 (II)
in which
R1 and R2 denote, independently of each other, linear C12 to C24 alkyl groups;
X denotes an oxygen atom, a sulphur atom or a sulphoxide or methylene group;
Y denotes an oxygen atom, a sulphur atom or a sulphoxide or methylene group;
when Y denotes a methylene group, the sum of the number of carbon atoms present in the groups R1 and R2 has a value ranging from 24 to 44 and preferably from 28 to 40 inclusive;
when Y does not denote a methylene group, the sum of the carbon atoms present in the groups R1 and R2 has a value ranging from 24 to 44 and preferably from 28 to 40 inclusive;
when X or Y denotes sulphoxide, Y or X does not denote sulphur;
C) acyl derivatives.

3. Composition according to either of Claims 1 and 2, **characterized in that** the pearlescent agents and/or opacifiers are chosen from:
A) distearyl ether,
B) compounds of formula (II) for which X denotes oxygen, Y denotes methylene and R1 and R2 denote radicals containing 12 to 22 carbon atoms,
C) ethylene glycol distearate.

4. Composition according to any one of Claims 1 to 3, **characterized in that** the saturated linear fatty alcohol containing 22 carbon atoms represents from 0.5% to 10% by weight, preferably from 0.5% to 5% by weight and even more preferentially from 0.5% to 3% by weight, relative to the total weight of the final composition.

5. Composition according to any one of Claims 1 to 4, **characterized in that** the saturated linear fatty alcohol containing 18 carbon atoms represents from 0.3% to 10% by weight, preferably from 0.5% to 5% by weight and even more preferentially from 0.5% to 3% by weight, relative to the total weight of the final composition.

6. Composition according to any one of Claims 1 to 5, **characterized in that** the C18 fatty alcohol/C22 fatty alcohol ratio is between 0.2 and 20, especially between 0.25 and 10 and preferably between 0.3 and 5.

7. Composition according to any one of Claims 1 to 6, **characterized in that** the opacifier and/or pearlescent agent represents from 0.5% to 15% by weight and preferably from 1% to 5% by weight, relative to the total weight of the final composition.

8. Composition according to any one of Claims 1 to 7, **characterized in that** the surfactant base is present at between 1% and 60% by weight, preferably between 3% and 40% and even more preferentially between 5% and 30%, relative to the total weight of the composition.

9. Composition according to any one of the preceding claims, **characterized in that** it also comprises at least one conditioner.

10. Composition according to the preceding claim, **characterized in that** the conditioner is chosen from poly-α-olefins, fluoro oils, fluoro waxes, fluoro gums, carboxylic acid esters, silicones, cationic polymers, mineral, plant or animal oils, ceramides and pseudoceramides, and mixtures thereof.

11. Composition according to the preceding claim, **characterized in that** the conditioners represent from 0.001% to 10% by weight, preferably from 0.005% to 5% by weight and even more preferentially from 0.01% to 3% by weight, relative to the total weight of the composition.

12. Composition according to any one of the preceding claims, **characterized in that** it is in the form of a gel, a milk, a cream, a more or less thickened lotion or a mousse.

13. Composition according to any one of the preceding claims, **characterized in that** it constitutes a foaming detergent composition such as shampoos, shower gels and bubble baths.

14. Use of at least one saturated linear fatty alcohol with a long chain containing 22 carbon atoms, at least one saturated linear fatty alcohol with a long chain containing 18 carbon atoms and at least one opacifier and/or pearlescent agent, the C18 fatty alcohol/C22 fatty alcohol ratio being greater than 0.15, as a suspension agent for a conditioner which is insoluble in a cosmetic composition, in particular a foaming conditioning and washing composition containing, in a cosmetically acceptable aqueous medium, a surfactant base.

15. Use of at least one saturated linear fatty alcohol with a long chain containing 22 carbon atoms and at least one saturated linear fatty alcohol with a long chain containing 18 carbon atoms, the C18 fatty alcohol/C22 fatty alcohol ratio being greater than 0.15, to give a pearlescent effect to, and/or to improve the pearlescent effect of, compositions comprising at least one opacifier and/or pearlescent agent and optionally at least one surfactant base.

16. Use of at least one saturated linear fatty alcohol with a long chain containing 22 carbon atoms, at least one saturated linear fatty alcohol with a long chain containing 18 carbon atoms, the C18 fatty alcohol/C22 fatty alcohol ratio being greater than 0.15, and an opacifier and/or pearlescent agent, as a pearlescent agent.

17. Cosmetic process for treating keratin materials, in particular the hair, **characterized in that** it consists in applying to said materials a composition as defined in any one of Claims 1 to 13, optionally followed by rinsing with water.
